# EUROPÄISCHE PATENTSCHRIFT

(11) **EP 0 533 726 B1**
(45) Veröffentlichungstag und Bekanntmachung des Hinweises auf die Patenterteilung: **30.11.1994**
(21) Anmeldenummer: 91910606.2
(22) Anmeldetag: 06.06.1991
(51) Int. Cl.: C07C 53/126, C07C 51/41, C08K 5/09, A23K 1/16

(54) **VERFAHREN ZUR HERSTELLUNG VON METALLSEIFEN**
PROCESS FOR PRODUCING METAL SOAPS
PROCEDE DE PRODUCTION DE SAVONS METALLIQUES

(30) Priorität: 15.06.1990 DE 4019167
(43) Veröffentlichungstag der Anmeldung: 31.03.1993
(73) Patentinhaber: Henkel Kommanditgesellschaft auf Aktien, 40191 Düsseldorf (DE)
(72) Erfinder: WÜST, Willi, D-4030 Ratingen 6 (DE); DÜRR, Gottfried, D-4000 Düsseldorf 13 (DE); WOLLMANN, Josef, D-5120 Herzogenrath 3 (DE); LIEBS, Harald, D-5090 Leverkusen (DE); Scheck,Hans, W-2854 Loxstedt 1 (DE)
(86) Internationale Anmeldenummer: EP9101051
(87) Internationale Veröffentlichungsnummer: WO9119691

(56) Entgegenhaltungen:
- EP-A- 0 304 831
- EP-A- 0 330 097
- EP-B- 0 163 395

## Beschreibung

Die Erfindung betrifft ein Verfahren zur Herstellung von festen, neutralen oder basischen Metallseifen durch eine kontrollierte Fest-Flüssig-Reaktion von flüssigen Fettsäuren mit festen Metalloxiden und/oder Metallhydroxiden in einer externen Vormischzone unter vermindertem Druck.

Es ist bekannt, daß man die Herstellung von Metallseifen entweder nach dem Schmelzverfahren durch Umsetzung der geschmolzenen Fettsäure mit Metalloxiden, -hydroxiden und/oder geeigneten Metallsalzen oder nach dem Fällungsverfahren durch doppelte Umsetzung ihrer Natriumseifen mit wasserlöslichen Salzen der entsprechenden Metalle durchführt. Das Fällungsverfahren liefert im allgemeinen sehr saubere und voluminöse Produkte, die bei Temperaturen unterhalb von 100 ° verarbeitet werden können. Es ist aber durch Filtrations- und Trocknungskosten wesentlich aufwendiger als das Schmelzverfahren.

Für viele technische Zwecke, wie beispielsweise als Stabilisator bei der Verarbeitung von Kunststoffen, genügen hinsichtlich der Reinheit und Farbe die nach dem Schmelzverfahren hergestellten Stabilisatoren. Als entsprechend wirkende Stabilisatoren haben sich beispielsweise die Blei-, Barium- und Cadmiumverbindungen in der Praxis bewährt. Diese Substanzen wirken jedoch stark toxisch, insbesondere wenn sie in Pulverform vorliegen. Für den Hersteller von pulverförmigen Stabilisatoren stellt insbesondere die sich bei der Verarbeitung bildende Staubentwicklung eine besondere Gefährdung dar, da die pulverförmigen Metallseifen durch die Atmungsorgane aufgenommen werden und dort ihre toxische Wirkung entfalten können.

In den deutschen Patentschriften DE-PS 15 44 697 und DE-PS 17 94 429 werden aus diesen Gründen, insbesondere Schmelzverfahren offenbart, die zur Verhinderung der Staubbildung eine Stabilisator-Gleitmittel-Kombination beanspruchen, die aus einem in der Schmelze vereinigten Gemisch einer als Gleitmittel geeigneten rein organischen Komponente, wie beispielsweise die Ester aus Wachssäuren und höheren aliphatischen Alkoholen, Paraffine oder Fettalkohole, aus einer geeigneten Metallseife, aus einer langkettigen aliphatischen Carbonsäure oder aus basischen Bleisalzen anorganischer oder organischer Säuren besteht. Insbesondere aus der Lehre der DE-PS 15 45 697 ist ein Verfahren zur Herstellung dieser staubfreien Stabilisator-Gleitmittel-Kombinationen bekannt, in welchem die toxischen, nichtlöslichen, staubförmigen Stabilisatoren in den Schmelzen der Gleitmittel oder den geschmolzenen Gemischen von Stabilisatoren und Gleitmitteln dispergiert und mit Hilfe einer Schuppenwalze oder durch einfaches Auslaufen in Pfannen in den festen Zustand überführt werden. Die pulverförmigen toxischen Stabilisatoren werden durch diesen Vorgang vollständig von den nichttoxischen Gleitmitteln umhüllt. Sie liegen in fester Form vor und sind völlig staubfrei, da die Gleitmittel von Natur aus eine wesentlich bessere Haftfähigkeit besitzen als die pulverförmigen Stabilisatoren.

Ferner sind aus der europäischen Offenlegungsschrift EP-A 163 395 und der britischen Offenlegungsschrift GB-A 21 13 521 Verfahren zur Herstellung von Futtermitteln bekannt, bei denen flüssige bzw. geschmolzene Fettsäuren in Gegenwart von Proteinen und Kohlehydraten mit Calciumoxid bzw. Calciumhydroxid umgesetzt werden. Diese Verfahren sind apparativ jedoch sehr aufwendig, da das offenbar noch nicht vollständig abreagierte Reaktionsgemisch zur Nachreaktion und Trocknung auf einem endlosen Band oder dergleichen ausgebreitet werden muß. Bei dieser Arbeitsweise dürften die erhaltenden Calciumseifen beim Weglassen der genannten Zusätze wie Proteine oder Kohlenhydrate, nur schwer in die Form freifließender Teilchen zu bringen sein.

Aus der Lehre der deutschen Offenlegungsschrift DE-OS 38 06 192 der Anmelderin ist schließlich ein Verfahren zur Herstellung von pulverförmigen basischen Metallseifen bekannt, bei dem pulverförmige Fettsäuren mit pulverförmigen Metalloxiden bzw. Metalloxidgemischen in Gegenwart von Wasser bzw. einer Säure als Katalysator bei Temperaturen von Umgebungstemperatur bis 100 °C, gegebenenfalls unter vermindertem Drucken umgesetzt werden, wobei das Reaktionsgemisch während der gesamten Umsetzung in Form von diskreten, freifließenden Teilchen vorliegen soll.

Die Aufgabe der vorliegenden Erfindung besteht nunmehr darin, ein verbessertes Verfahren zur Herstellung von pulverförmigen, neutralen oder basischen Metallseifen zur Verfügung zu stellen, das eine staubfreie, umweltfreundliche Dosierung des festen Metalloxids bzw. Metallhydroxids in die Fettsäureschmelze in Form einer kontrollierten Fest-Flüssig-Reaktion ohne die Zugabe eines Katalysators ermöglicht. Eine weitere Aufgabe der vorliegenden Erfindung liegt darin, ein Verfahren zur Aufarbeitung der flüssigen Metallseifen zu entwickeln, welches das entstehende Neutralisationswasser unter Verfahrensbedingungen über eine geeignete Kondensationsvorrichtung auf einfache Weise abführt.

Die technische Lösung der erfindungsgemäßen Aufgabe geht von dem Konzept aus, Anteile der vorgelegten, fließfähigen Phase der Fettsäuremischung aus dem Reaktorinhalt unter Verfahrensbedingungen abzuführen und einer Vormischphase zuzuführen, in die das feste Metalloxid und/oder Metallhydroxid eindosiert wird. In dieser Vormischzone wird aus der im Kreislauf geführten Flüssigphase und der zudosierten Feststoffphase eine Abmsichung gebildet, deren Beschaffenheit so eingestellt wird, daß die Abmischung als sogenannte "lebende Dichtung" fungieren kann. Durch die Auswahl geeigneter Reaktionsbedingungen für die einzudosierenden Feststoffreaktanten in das unter vermindertem Druck gehaltene Reaktorinnere ist der Druck im Reaktorinneren unter Beachtung der Schaumentwicklung zuverlässig steuerbar. Nach Abschluß der Eindosierung des Feststoffes kann der Reaktionsraum der Vormischzone durch geeignete mechanische Elemente verschlossen werden und damit die Einführung von Außenluft in das Reaktionssystem vermieden werden.

Gegenstand der vorliegenden Verbindung ist daher ein Verfahren zur Herstellung von festen, neutralen oder basischen Metallseifen der allgemeinen Formel

M (R-COO)(R¹-COO)

in der M ein oder mehrere Metallkationen aus der von Ca, Mg, Cd, Ba, Zn und Pb gebildeten Gruppe und R bzw. R¹ gleich oder verschieden geartete Kohlenwasserstoffreste mit 8 bis 34 C-Atomen bedeuten,
durch Direktsynthese aus einer entsprechenden Fettsäure oder Fettsäuremischung mit Metalloxiden und/oder Metallhydroxiden, dadurch gekennzeichnet, daß man einen Teilstrom der im Reaktor unter vermindertem Druck gehaltenen, flüssigen Phase einer Fettsäure oder Fettsäuremischung in eine externe Vormischzone abzieht, in der Vormischzone mit einem festen Metalloxid und/oder Metallhydroxid in Form einer Fest-Flüssig-Reaktion in Kontakt bringt, das gebildete Umsetzungsprodukt, über einen nachgeschalteten Intensivmischer in den Reaktor zurückführt und das gebildete Neutralisationswasser über die Gasphase kontinuierlich aus dem Reaktor abführt.

Die Vorteile des erfindungsgemäßen Verfahrens liegen einerseits in der exakten, staubfreien und dementsprechend umweltfreundlichen Dosierung der Metalloxide bzw. Metallhydroxide in die Fettsäureschmelze, wodurch infolge geringerer Schaumbildung mit einer höheren Reaktorauslastung gearbeitet werden kann und andererseits darin, daß auf den Zusatz eines Katalysators nach dem üblichen Stand der Technik verzichtet werden kann. Als ein weiterer Vorteil des erfindungsgemäßen Verfahrens erweist sich die Erkenntnis, daß die in das Reaktionsinnere zurückgeführten Metallseifen keiner zeitaufwendigen Nachreaktion unterzogen werden müssen, da die Neutralisationsreaktion bereits vollständig in der externen Reaktionsschleife abgelaufen ist.

In einer bevorzugten Ausführungsform der vorliegenden Erfindung führt man die erfindungsgemäße Fest-Flüssig-Reaktion in der Weise durch, daß die externe Vormischzone vor dem Intensivmischer mit dem verminderten Druck des Reaktorinneren und gleichzeitig über die Fördervorrichtung für die Feststoffe mit dem Umgebungsdruck in unmittelbarem Druckausgleich steht und die Konsistenz der in der Vormischzone gebildeten, pastenförmigen Abmischung derart gewählt wird, daß die Abmischung als Dichtmasse zum Druckausgleich dient.

Im Sinne der vorliegenden Erfindung führt man die Schmelze der Fettsäure oder Fettsäuremischung unter einem verminderten Reaktorinnendruck von etwa 100 bis 900 mbar, vorzugsweise 200 bis 800 mbar, in die externe Vormischzone ab, wobei der Vormischzoneninnendruck unter Berücksichtigung des Druckabfalles abgemindert sein sollte. In der Vormischzone wird die Fettsäureschmelze, gegebenenfalls mehrfach mit wenigstens stöchiometrischen Mengen an Metalloxid und/oder Metallhydroxid, in Form einer Fest-Flüssig-Reaktion unter vermindertem Druck in Kontakt gebracht.

Zwischen dem Reaktorinneren und dem atmosphärischen Außendruck, besteht dementsprechend eine beträchtliche Druckdifferenz. Die als "lebende Dichtung" fungierende Abmischung in der Vormischzone muß in ihrer Beschaffenheit also so eingestellt werden, daß sie unter jeweils gegebenen Verhältnissen als wirkliches Abdichtelement gegen den Einbruch des weitaus höheren Außendruckes in das Reaktorinnere dienen kann. Dabei ist diese Gefährdung des angestrebten Durckausgleichs nicht die einzige Belastung der pastenförmigen Dichtmasse. Zur Herstellung der Abmischung muß der zugeführte Flüssiganteil in die Vormischzone mit beschränkt erhöhtem Druck eingespeist werden. Hierbei besteht die Gefahr, daß bei zu hohen Flüssigkeitsdrucken Anteile der Flüssigphase durch das von "außen", vorzugsweise kontinuierlich, zudosierte pulverförmige Metalloxid und/oder Metallhydroxid durchbrechen. Hier liegt also eine zweite Gefahrenquelle für den angestrebten störungsfreien, bevorzugt kontinuierlichen Verfahrensabschnitt der zeitlich gesteuerten Eindosierung der Feststoffreaktanten in das Reaktorinnere.

Zum Ausschluß von Störungen der zuletzt dargestellten Art sieht das erfindungsgemäße Verfahren in der bevorzugten Ausführungsform vor, daß auch in der Vormischzone bei Unterdruck gearbeitet wird. Die hier eingestellten Unterdrucke sind aber im Vergleich mit dem im Reaktorinneren vorherrschenden Unterdruck geringer. Bevorzugt wird der Druck im Bereich der Vormischzone bei etwa 400 bis 900 mbar und insbesondere im Bereich von etwa 750 bis 850 mbar gehalten. Die Einstellung dieses Druckbereiches gelingt durch angepaßte Steuerung der flüssigen und festen Materialströme zur Vormischzone und deren Abführung in das Reaktorinnere. Zur Ausbildung der in der Vormischzone als lebende Dichtung fungierenden Abmischung haben sich ausgewählte Massenverhältnisse der Ströme von flüssigen und festen Reaktanten als vorteilhaft erwiesen. Die bevorzugten Massenverhältnisse der Flüssigphase (m₁) zu der Masse des eingeführten Feststoffreaktanten (m₂) liegen im Bereich von etwa 20 : 1 bis 100 : 1. Als besonders geeignet haben sich Werte im Bereich von etwa 30 : 1 bis 80 : 1 für diesen Massenquotienten m₁/m₂ erwiesen.

Bereits in der eingangs genannten älteren Anmeldung der Anmelderin DE-OS-38 06 192 wird darauf verwiesen, daß bei der Umsetzung die Teilchen in diskreter, freifließender Form vorliegen sollen. Es wird daher vorgeschlagen - und davon macht auch die Lehre der Erfindung in ihrer bevorzugten Ausführungsform Gebrauch - die primär entstehende Wirkstoffmischung durch geeignete technische Reinstmischelemente einer Feindispergierung zu unterwerfen. Als besonders geeignet haben sich hier sogenannte Inline-Mischer, beispielsweise vom Stator/Rotor-Prinzip, bewährt. Im einzelnen ist dann die durch das erfindungsgemäße Verfahren betroffene Einheit der Reaktoranlage beispielsweise wie folgt ausgebildet:
Von der Hauptreaktion abgetrennt, aber in unmittelbarer Durckanbindung an die Hauptreaktionszone, ist eine Vormischzone vorgesehen. Dieser Vormischzone wird einerseits der trockene, bevorzugt pulverförmige, Feststoffreaktant, beispielsweise durch eine Förderschnecke, zugeführt. Gleichzeitig wird über eine Umlaufleitung aus dem Inneren des Reaktors Flüssigphase in Abmischung mit der eingespeisten festen Reaktantenmenge so der Vormischzone zugegeben, daß hier die Abmischung als lebende Dichtung - insbesondere im Bereich der angegebenen Massenverhältnisse von Flüssigphase zu Feststoffphase - ausgebildet wird. Aus der Vormischzone wandert die Paste im Sinne des Druckgefälles in Richtung auf das Reaktorinnere in den Intensivmischer, beispielsweise also in den nachgeschalteten Inline-Mischer, und tritt aus diesem dann in das Reaktorinnere ein. Die Geschwindigkeit des Materialdurchtritts durch die Vormischzone kann unter diesen Bedingungen praktisch beliebig gesteuert und dem Reaktionsablauf im Reaktorinneren optimal angepaßt werden. Es ist eine absatzweise und/oder kontinuierliche Zugabe des Feststoffreaktanten möglich.

Nach der Dosierung der Feststoffreaktanten wird der Reaktionsinnenraum von der Umgebung abgeschlossen. Möglich ist das beispielsweise durch ein in der Vormischzone vorgesehenes Trennorgan, das die Vormischzone und die Förderschnecke für das Feststoffgut voneinander trennt. Während der Einspeisung der Feststoffreaktanten ist das Trennorgan geöffnet. Nach Zugabe der insgesamt benötigten Menge des Feststoffreaktanten wird es wieder geschlossen.

In einer besonders bevorzugten Ausführungsform dieser Erfindung erfolgt die Zudosierung des festen Metalloxids bzw. Metallhydroxids in die Fettsäureschmelze mit Hilfe eines Inline-Mischers, welcher das Fettsäuregemisch mit einer Leistung von etwa 1 bis 20 m³ pro Stunde und die Feststoffe mit einer Leistung von 50 kg bis 500 kg pro Stunde, vorzugsweise 100 bis 300 kg/h, dosieren. Um eine maximale Förderleistung des Inline-Mischers zu gewährleisten muß der Anlagendruck so variiert werden, daß die Fettsäureschmelze nicht in den Raum der Feststoffförderschnecke zurückfließt.

Im Sinne dieser Erfindung wird dabei das feste Metalloxid und/oder Metallhydroxid in die Fettsäureschmelze in der Weise zudosiert, daß die Teilchengröße des gebildeten Agglomerats unterhalb von 10 µm liegt und die Säurezahlen der gebildeten Metallseifen zwischen 0,1 und 30, vorzugsweise zwischen 0,5 und 10 liegen.

Nach dem erfindungsgemäßen Verfahren lassen sich sämtliche Fettsäuren mit 8 bis 34 C-Atomen natürlichen oder synthetischen Ursprungs in die gewünschten, pulverförmigen neutralen oder basischen Metallseifen überführen, wobei bevorzugt gesättigte Fettsäuren eingesetzt werden. Besonders vorteilhaft läßt sich das erfindungsrelevante Verfahren für die Umsetzung von technischen Gemischen natürlicher, gesättigter Fettsäuren mit 12 bis 22 C-Atomen mit der Maßgabe einsetzen, daß der Schmelzpunkt der Fettsäuren unterhalb von 200 °C, vorzugsweise unterhalb von 150 °C, liegt. Typische Beispiele für derartige Fettsäuren sind Palmitin-, Stearin-, Behen- und Montansäuren sowie technische Gemische die reich an den genannten Fettsäuren sind.

Im Sinne dieser Erfindung können als Metalloxide bzw. Metallhydroxide die dem Fachmann bekannten Oxide und/oder Hydroxide von Calcium, Magnesium, Cadium, Barium, Zink und/oder Blei eingesetzt werden. Erfindungsgemäß wird Calciumhydroxid, Magnesiumoxid und/oder Zinkoxid bevorzugt eingesetzt. Prinzipiell können natürlich auch andere Salze der oben angeführten Metallionen, wie beispielsweise die Carbonate, Acetate, Stearate oder andere Metallseifen zur Anwendung im erfindungsgemäßen Verfahren kommen.

In einer bevorzugten Ausführungsform der vorliegenden Erfindung werden zur Viskositätsregulierung, insbesondere bei Fettsäuren mit einem Schmelzpunkt oberhalb von 100 bis 150 °C, auf die Metallseifen abgestimmte Verdünnungsmittel zugefügt. Geeignete Verdünnungsmittel sind beispielsweise Paraffine mit einem Schmelzpunkt von 20 bis 150 °C, vorzugsweise 60 bis 100 °C, Ester von Wachssäuren und höheren aliphatischen Fettalkoholen mit vorzugsweise 12 bis 22 C-Atomen, Walrat oder geeignete Fettsäuren. Die erfindungsgemäßen Verdünnungsmittel können je nach Metallseife in einem Gewichtsverhältnis von 1 : 10 bis 10 : 1, vorzugsweise 1 : 2 bis 2 : 1, mit den Ausgangsfettsäuren gemischt werden, wobei dieses Gewichtsverhältnis von der Pumpfähigkeit des Reaktorinhaltes abhängig ist.

Um einen kontinuierlichen Betrieb des Feeders zu gewährleisten, hat es sich im Rahmen der Erfindung als empfehlenswert erwiesen, die Schnecke mit einer antiadhäsiven Beschichtung zu überziehen. Die Beschichtung soll eine Haftung oder Brückenbildung des Metalloxids bzw. Metallhydroxids auf der Förderschnecke verhindern.

Als weitere Optimierung des erfindungsgemäßen Verfahrens hat sich die Verwendung einer Einspritzkühlung im Kondensationssystem bewährt. In dem Kondensationssystem wird der überhitzte Wasserdampf aus der Neutralisationsreaktion über eine Einspritzdüse, die mit kaltem Brauchwasser betrieben wird, abgekühlt. Mit dieser Einspritzkühlung wird die Gefahr von Inkrustierungen des Kondensationssystemes nahezu unterdrückt, und die Einstellung des Anlagendruckes frei wählbar gehalten.

Die Erfindung wird im folgenden anhand bevorzugter Ausführungsbeispiele näher erläutert.

### Beispiele

### Beispiel 1

In einem 2,5 m³ - Reaktionsbehälter, der mit einem vierstufigen MIG-Rührwerk mit bodenständigem Rührer und einem Kondensationssystem ausgerüstet ist, wurden bei geschlossenem Bodenablaßventil 468 kg technische Stearinsäure vorgelegt und die Mantelheizung in Betrieb genommen. Bei einer Temperatur von etwa 70 °C wurden 411 kg Glycerindistearat zugeführt und das Reaktionsgemisch auf 150 °C unter Rühren erwärmt. Gleichzeitig erfolgte eine Reduzierung des Anlagendrucks auf 550 mbar. Anschließend wurde nach Öffnen des Bodenablaßventils die Kreislaufpumpe der externen Reaktionsschleife und der Inline-Mischer in Betrieb genommen. Durch Inbetriebnahme der Dosierschnecke und Öffnen des Dichtorgans wurden innerhalb von 45 Minuten 63 kg Calciumhydroxid in die Vormischzone eindosiert. Hierbei wurde die Förderleistung des Inline-Mischers so eingestellt, daß einerseits keine übermäßige Schaumbildung durch die Zurückführung der Metallseife über den Inline-Mischer im Reaktionsbehälter eintrat und andererseits keine Fettsäure in die Dosierschnecke zurückfloß. Nach Beendigung der Feststoffdosierung wurde der Anlagendruck auf etwa 30 mbar reduziert und das Neutralisationswasser kontinuierlich über das Kondensationssystem abgeführt. Durch kontinuierliche Probenahme und Bestimmung der Säurezahl (Soll: 5 bis 10) wurde der Endpunkt der Reaktion (ca. 1 Stunde) bestimmt. Anschließend wurde der Anlagendruck mit Stickstoff auf Normaldruck angehoben und das flüssige Reaktionsgemisch in üblicher Weise, z. B. durch Verprillen, konfektioniert. Der Schmelzpunkt der entstandenen Metallseife lag bei einer 99 %-igen Ausbeute bei etwa 95 °C.

### Beispiel 2

In einen 10 m³-Reaktionsbehälter, der wie in Beispiel 1 ausgerüstet war, wurden 2800 kg technische Stearinsäure vorgelegt und auf etwa 95 °C erwärmt. Anschließend wurden innerhalb einer Stunde 2100 kg eines Paraffinwachses (Schmelzpunkt: < 80 °C) mit der Maßgabe zudosiert, daß eine Temperatur von 85 ° nicht überschritten wurde. Nach der Erhöhung der Reaktionstemperatur auf 150 °C wurde der Anlagendruck auf 550 mbar gesenkt und über die Kreislaufpumpe und den Inline-Mischer die externe Reaktionsschleife in Betrieb genommen. Anschließend wurden 160 kg Zinkoxid in dem Inline-Mischer mittels Dosierschnecke innerhalb von 45 Minuten zudosiert. Übergangs los wurden in gleicher Weise innerhalb von 35 Minuten 213 kg Calciumhydroxid zudosiert, wobei der Unterdruck des Gesamtsystems nicht beeinträchtigt wurde. Nach Beendigung der Festkörperdosierung wurde der Anlagendruck auf etwa 30 mbar reduziert und das Neutralisationswasser kontinuierlich über das Kondensationssystem abgeführt. Durch kontinuierliche Probenahme und Bestimmung der Säurezahl (Soll: 5 bis 10) wurde der Endpunkt der Reaktion (ca. 1 Stunde) bestimmt. Anschließend wurde der Anlagendruck mit Stickstoff auf Normaldruck angehoben und das flüssige Reaktionsgemisch in üblicher Weise, z. B. durch Verprillen, konfektioniert. Der Schmelzpunkt der entstandenen Metallseife lag bei einer 99 %-igen Ausbeute bei etwa 100 °C.

## Patentansprüche

1. Verfahren zur Herstellung von festen, neutralen oder basischen Metallseifen der allgemeinen Formel
**M (R-COO) (R¹-COO)**
in der M ein oder mehrere Metallkationen aus der von Ca, Mg, Cd, Ba, Zn und Pb gebildeten Gruppe und R bzw. R¹ gleich oder verschieden geartete Kohlenwasserstoffreste mit 8 bis 34 C-Atomen bedeuten,
durch Direktsynthese aus einer entsprechenden Fettsäure oder Fettsäuremischung mit Metalloxiden und/oder Metallhydroxiden, dadurch gekennzeichnet, daß man einen Teilstrom der im Reaktor unter vermindertem Druck gehaltenen, flüssigen Phase einer Fettsäure oder Fettsäuremischung in eine externe Vormischzone abzieht, in der Vormischzone mit einem festen Metalloxid und/oder Metallhydroxid in Form einer Fest-Flüssig-Reaktion in Kontakt bringt, das gebildete Umsetzungsprodukt über einen nachgeschalteten Intensivmischer in den Reaktor zurückführt und das gebildete Neutralisationswasser über die Gasphase kontinuierlich aus dem Reaktor abführt.

2. Verfahren nach Anspruch 1, dadurch gekennzeichnet, daß man die Fest-Flüssig-Reaktion in der Weise durchführt, daß die Vormischzone vor dem Intensivmischer mit dem verminderten Druck des Reaktorinneren und gleichzeitig über die Fördervorrichtung für die Feststoffe mit dem Umgebungsdruck in unmittelbarem Druckausgleich steht und die Konsistenz der in der Vormischzone gebildeten pastenförmigen Abmischung derart gewählt wird, daß diese Abmischung als Dichtmasse zum Druckausgleich dient.

3. Verfahren nach den Ansprüchen 1 und 2, dadurch gekennzeichnet, daß mit einem Reaktorinnendruck im Bereich von 100 bis 900 mbar, vorzugsweise 200 bis 800 mbar, gearbeitet wird.

4. Verfahren nach den Ansprüchen 1 bis 3, dadurch gekennzeichnet, daß man mit einem gegenüber dem Reaktorinnendruck abgemilderten Vormischzoneninnendruck von 400 bis 900, vorzugsweise 750 bis 850 mbar, arbeitet.

5. Verfahren nach den Ansprüchen 1 bis 4, dadurch gekennzeichnet, daß in der Vormischzone ein Massenverhältnis der Flüssigphase zur Feststoffphase im Bereich von 20 : 1 bis 100 : 1, vorzugsweise 30 : 1 bis 80 : 1, eingestellt wird.

6. Verfahren nach den Ansprüchen 1 bis 5, dadurch gekennzeichnet, daß die in der Vormischzone gebildete und als lebende Dichtung dienende Abmischung über einen Inline-Mischer in den Reaktorinnenraum zurückgeführt wird.

7. Verfahren nach den Ansprüchen 1 bis 6, dadurch gekennzeichnet, daß das feste Metalloxid und/oder Metallhydroxid der Vormischzone mit einer Förderschnecke zudosiert wird.

8. Verfahren nach den, Ansprüchen 1 bis 7, dadurch gekennzeichnet, daß man als Fettsäuren ein technisches Gemisch natürlicher Fettsäuren mit 8 bis 34, vorzugsweise 12 bis 22 C-Atomen mit der Maßgabe einsetzt, daß der Schmelzpunkt der Fettsäuren unterhalb von 200 °C, vorzugsweise unterhalb von 150 °C, liegt.

9. Verfahren nach den Ansprüchen 1 bis 8, dadurch gekennzeichnet, daß man als festes Metalloxid und/oder Metallhydroxid Calciumhydroxid, Magnesiumoxid und/oder Zinkoxid, einsetzt.

10. Verfahren nach den Ansprüchen 1 bis 9, dadurch gekennzeichnet, daß man zur Viskositätsregulierung, insbesondere bei Fettsäuren mit einem Schmelzpunkt oberhalb von 100 bis 150 °C, geeignete Verdünnungsmittel einsetzt.

11. Verfahren nach den Ansprüchen 1 bis 10, dadurch gekennzeichnet, daß man die Verdünnungsmittel, vorzugsweise Paraffine mit einem Schmelzpunkt von 20 bis 150 °C, insbesondere 60 bis 100 °C, mit den entsprechenden Ausgangsfettsäuren, in einem Gewichtsverhältnis von 1 : 10 bis 10 : 1, vorzugsweise 1 : 2 bis 2 : 1, einsetzt.

## Claims

1. A process for the production of solid, neutral or basic metal soaps corresponding to the following general formula
**M (R-COO)(R¹-COO**
**)**
in which M represents one or more metal cations from the group consisting of Ca, Mg, Cd, Ba, Zn and Pb and R and R¹ may be the same or different and represent C₈₋₃₄ hydrocarbon radicals,
by direct synthesis from a corresponding fatty acid or fatty acid mixture with metal oxides and/or metal hydroxides, characterized in that a stream of the liquid phase of a fatty acid or fatty acid mixture kept under reduced pressure in the reactor is run off into an external premixing zone and contacted therein with a solid metal oxide and/or metal hydroxide in a solid/liquid reaction, the reaction product formed is returned to the reactor via a following intensive mixer and the water of neutralization formed is continuously removed from the reactor via the gas phase.

2. A process as claimed in claim 1, characterized in that the solid/liquid reaction is carried out in such a way that the premixing zone before the intensive mixer is in pressure equalization with the reduced pressure prevailing inside the reactor and at the same time - via the feed unit for the solids - with the ambient pressure and the consistency of the paste-form mixture formed in the premixing zone is selected so that this mixture serves as a sealing compound for pressure equalization.

3. A process as claimed in claims 1 and 2, characterized in that the internal reactor pressure is in the range from 100 to 900 mbar and preferably in the range from 200 to 800 mbar.

4. A process as claimed in claims 1 to 3, characterized in that the premixing zone is operated at a reduced internal pressure in relation to the internal reactor pressure of 400 to 900 and preferably 750 to 850 mbar.

5. A process as claimed in claims 1 to 4, characterized in that a ratio by weight of the liquid phase to the solid phase of 20:1 to 100:1 and preferably 30:1 to 80:1 is established in the premixing zone.

6. A process as claimed in claims 1 to 5, characterized in that the mixture formed in the premixing zone and serving as a living seal is returned to the interior of the reactor via an inline mixer.

7. A process as claimed in claims 1 to 6, characterized in that the solid metal oxide and/or metal hydroxide is delivered to the premixing zone by a screw conveyor.

8. A process as claimed in claims 1 to 7, characterized in that a technical mixture of natural fatty acids containing 8 to 34 carbon atoms and preferably 12 to 22 carbon atoms is used for the fatty acids, with the proviso that the melting point of the fatty acids is below 200°C and preferably below 150°C.

9. A process as claimed in claims 1 to 8, characterized in that calcium hydroxide, magnesium oxide and/or zinc oxide is used as the solid metal oxide and/or metal hydroxide.

10. A process as claimed in claims 1 to 9, characterized in that suitable diluents are used to regulate viscosity, particularly where fatty acids having a melting point above 100 to 150°C are used.

11. A process as claimed in claims 1 to 10, characterized in that the diluents, preferably paraffins having a melting point of 20 to 150°C and, more particularly, 60 to 100°C, are used with the corresponding starting fatty acids in a ratio by weight of 1:10 to 10:1 and preferably 1:2 to 2:1.

## Revendications

1. Procédé de préparation de savons métalliques solides, neutres ou basiques de formule générale :
M(R-COO)(R¹-COO)
dans laquelle M représente un ou plusieurs cations métalliques du groupe constitué de Ca, Mg, Cd, Ba, Zn et Pb et R ou R¹ représentent des radicaux hydrocarbure de 8 à 34 atomes de C, identiques ou différents,
par synthèse directe à partir d'un acide gras ou d'un mélange d'acides gras correspondant et d'oxydes métalliques et/ou d'hydroxydes métalliques, caractérisé en ce qu'on soutire un courant partiel de la phase liquide, maintenue sous pression réduite dans le réacteur, qui contient un acide gras ou un mélange d'acide gras et on le conduit dans une zone externe de prémélange, on le met en contact dans la zone de prémélange avec un oxyde métallique et/ou un hydroxyde métallique solide pour réaliser une réaction solide-liquide, on introduit le produit de réaction formé dans le réacteur, par l'intermédiaire d'un mélangeur énergique en aval et on élimine en continu l'eau de neutralisation formée via la phase gazeuse hors du réacteur.

2. Procédé selon la revendication 1, caractérisé en ce qu'on réalise la réaction solide-liquide, de sorte que la zone de prémélange se trouve en amont du mélangeur énergique en équilibre de pression direct avec la pression réduite de l'intérieur du réacteur et simultanément en équilibre avec la pression environnante par l'intermédiaire du dispositif d'introduction des solides et on choisit la consistance du mélange pâteux formé dans la zone de prémélange, de sorte que ce sous-mélange serve de mastic pour compenser la pression.

3. Procédé selon les revendications 1 et 2, caractérisé en ce qu'on travaille avec une pression interne de réacteur comprise entre 100 et 900 mbar, de préférence entre 200 et 800 mbar.

4. Procédé selon les revendications 1 à 3, caractérisé en ce qu'on travaille avec une pression interne de zone de prémélange atténuée par rapport à la pression interne du réacteur, comprise entre 400 et 900, de préférence entre 750 et 850 mbar.

5. Procédé selon les revendications 1 à 4, caractérisé en ce que dans la zone de prémélange, on ajuste une proportion massique de la phase liquide à la phase solide comprise dans l'intervalle de 20 : 1 à 100 : 1, de préférence 30 : 1 à 80 : 1.

6. Procédé selon les revendications 1 à 5, caractérisé en ce que l'on introduit à l'intérieur du réacteur par un mélangeur en ligne le sous-mélange formé dans la zone de prémélange et servant de joint actif.

7. Procédé selon les revendications 1 à 6, caractérisé en ce qu'on introduit en dosant l'oxyde métallique ou l'hydroxyde métallique solide dans la zone de prémélange avec une vis de transfert.

8. Procédé selon les revendications 1 à 7, caractérisé en ce qu'on utilise comme acides gras un mélange technique d'acides gras naturels de 8 à 34, de préférence 12 à 22 atomes de C, à la condition que le point de fusion des acides gras se trouve en dessous de 200°C, de préférence en dessous de 150°.

9. Procédé selon les revendications 1 à 8, caractérisé en ce qu'on utilise comme oxyde métallique et/ou hydroxyde métallique solides de l'hydroxyde de calcium, de l'oxyde de magnésium et/ou de l'oxyde de zinc.

10. Procédé selon les revendications 1 à 9, caractérisé en ce qu'on utilise un diluant approprié à l'ajustement de viscosité, notamment pour les acides gras ayant un point de fusion supérieur à l'intervalle de 100°C à 150°C.

11. Procédé selon les revendications 1 à 10, caractérisé en ce qu'on utilise les diluants de préférence des paraffines ayant un point de fusion compris entre 20 et 150°C notamment entre 60 et 100°C, avec les acides gras de départ correspondants, en une proportion pondérale de 1 : 10 à 10 : 1, de préférence 1 : 2 à 2 : 1.
